# FASCICULE DE BREVET EUROPEEN

(11) **EP 0 632 715 B1**
(45) Date de publication et mention de la délivrance du brevet: **24.04.1996**
(21) Numéro de dépôt: 93905457.3
(22) Date de dépôt: 08.03.1993
(51) Int. Cl.: A61K 7/06

(54) **COMPOSITION COSMETIQUE POUR LE MAINTIEN DE LA COIFFURE, CONTENANT UNE PROTEINE DE LAIT ET/OU UN HYDROLYSAT DE PROTEINE DE LAIT ET UN HYDROLYSAT DE KERATINE**
KOSMETISCHE ZUSAMMENSETZUNG ZUR ERHALTUNG DER FRISUR ENTHALTEND EIN MILCHPROTEIN UND/ODER EIN MILCHPROTEINHYDROLYSATE UND EIN HYDROLYSATE
COSMETIC HAIR-PERMING COMPOSITION CONTAINING A MILK PROTEIN AND/OR A MILK PROTEIN HYDROLYSATE AND A KERATIN HYDROLYSATE

(30) Priorité: 09.03.1992 FR 9202776
(43) Date de publication de la demande: 11.01.1995
(73) Titulaire: L'OREAL, F-75008 Paris (FR)
(72) Inventeur: DUPUIS, Christine, F-75018 Paris (FR); DUBIEF, Claude, F-78150 Le Chesnay (FR)
(74) Mandataire: Casalonga, Axel
(86) Numéro de dépôt international: FR9300223
(87) Numéro de publication internationale: WO9317656

(56) Documents cités:
- EP-A- 0 334 776
- WO-A-89/05629
- WO-A-91/14418
- GB-A- 2 041 963
- US-A- 4 195 077
- US-A- 4 275 748

## Description

La présente invention concerne une composition cosmétique destinée à être utilisée pour le maintien des cheveux, contenant une protéine de lait et/ou un hydrolysat de protéine de lait et un hydrolysat de kératine.

Des compositions à usage cosmétique cutané contenant des protéines de lait ou de la kératine ont été décrites. La demande de brevet européen EP 0 334 776 décrit une composition à usage cutané contenant comme principe actif du sérum de colostrum, cette composition présentant des propriétés hydradantes, stimulantes et régénératives.

La demande de brevet WO 89/5629 décrit une composition cosmétique comprenant entre autres composants de la caséine ou de la kératine, cette composition étant destinée à favoriser la pénétration des substances protéiques ou des amino-acides dans la peau ou les cellules de la peau.

Enfin, des hydrolysats de protéines ont déjà été utilisés en combinaison avec de la polyvinylpyrolidone dans des compositions pour améliorer le maintien des cheveux, ainsi que le décrit la demande de brevet WO 91 144 18.

Par ailleurs, une composition cosmétique associant un agent tensio-actif et une protéine modifiée chimiquement a été décrite dans le brevet US 4 195 077, cette association ayant pour but de protéger la kératine de l'action agressive du détergent.

En particulier, les protéines ou leur hydrolysat sont utilisés dans des compositions pour le maintien des cheveux car elles confèrent à la chevelure une bonne tenue, et contrairement aux compositions contenant d'autres protéines comme les kératines elles n'entraînent pas de phénomène de poissage des cheveux. Ces compositions présentent cependant l'inconvénient de provoquer un phénomène de poudrage au brossage des cheveux, ce qui donne un aspect inesthétique à la chevelure. D'autre part, les mousses de coiffage contenant des protéines de lait ou leur hydrolysat ont une stabilité et une expansion insuffisante.

La demanderesse a découvert, de façon surprenante, que les associations de certains hydrolysats de kératine avec des protéines de lait et/ou leurs hydrolysats, conféraient à la chevelure une bonne tenue sans provoquer de poissage ni de poudrage des cheveux.

De plus, les associations particulières de l'invention, lorsqu'elles sont utilisées dans des mousses de coiffage, présentent un effet de synergie sur les qualités de rigidité, de stabilité et d'expansion ainsi qu'une plus faible aération des mousses, par rapport aux composants de ces associations pris individuellement.

L'invention a donc pour objet une composition cosmétique pour le maintien des cheveux contenant au moins une protéine de lait et/ou un hydrolysat de protéine de lait et au moins un hydrolysat de kératine.

Un autre objet de l'invention est constitué par un procédé de traitement cosmétique des cheveux destiné à favoriser leur maintien, consistant à leur appliquer l'association d'au moins une protéine de lait et/ou un hydrolysat de protéine de lait et au moins un hydrolysat de kératine.

D'autres objets de l'invention apparaîtront à la lecture de la description et des exemples qui suivent.

La composition cosmétique, conforme à l'invention, est essentiellement caractérisée par le fait qu'elle contient dans un milieu cosmétiquement acceptable, au moins une protéine de lait et/ou un hydrolysat de protéine de lait et au moins un hydrolysat de kératine de poids moléculaire compris entre 100 et 200.000.

Les hydrolysats de kératine, conformes à l'invention, sont obtenus par hydrolyse de kératines animales ou humaines issues par exemple de matériaux tels que les cheveux, la laine, la peau, les poils, les soies, les plumes, les écailles, les sabots ou la corne. Ils peuvent être modifiés chimiquement et portent dans ce cas au bout de leur chaîne peptidique ou greffés sur leur chaîne peptidique des groupements ammonium quaternaires.

Parmi les hydrolysats de kératine utilisés selon la présente invention, on peut citer plus particulièrement :
- les hydrolysats de kératine obtenus par hydrolyse modérée de kératine de sabots de bovin d'un poids moléculaire d'environ 100.000, tel que le produit vendu sous la dénomination KERASOL par la Société CRODA;
- les hydrolysats de kératine obtenus par hydrolyse acide ménagée, d'un poids moléculaire d'environ 150, tel que le produit vendu à 25% de matière active sous la dénomination CROTEIN HKP S/F par la Société CRODA;
- les hydrolysats de kératine portant sur la chaîne peptidique des groupements ammonium quaternaires, comportant au moins une chaîne grasse en C₁₀-C₁₈ tel que le produit vendu sous la dénomination CROQUAT WKP par la Société CRODA.

Selon la présente invention, toutes les protéines de lait sont utilisables ainsi que leurs mélanges et leurs hydrolysats. Le lait renferme essentiellement deux types de protéines, les caséines et les protéines du lactosérum dont les principales sont l'α-lactalbumine, la β-lactoglobuline et les immunoglobulines.

Les protéines de lait utilisables selon l'invention sont obtenues de préférence à partir de lait de vache ou de sous-produits du lait résultant par exemple de la fabrication du fromage ou du beurre tel que le lactosérum ou le babeurre.

Ces protéines peuvent être concentrées et/ou séparées et séchées par diverses méthodes classiques.

On utilise plus particulièrement :
- les caséines obtenues à partir du lait écrémé par précipitation, par voie acide, par fermentation lactique ou par de la presure, puis séchées. On préfère utiliser des sels solubles de caséines obtenus par neutralisation, tels que les caséinates de sodium, de calcium ou de magnésium.

De tels produits sont vendus par exemple par les Sociétés DE MELKINDUSTRIE VEGHEL BV, ARMOR PROTEINES, UNION LAITIERE NORMANDE (U.L.N.);
- les protéines du lactosérum, séparées du lactosérum par divers procédés tels que l'ultrafiltration, l'électrodialyse, la chromatographie échangeuse d'ions, la chromatographie d'exclusion par la taille.

De tels produits sont vendus par exemple par la Société ARMOR PROTEINES sous la dénomination PROSERIC DF 90, par la Société U.L.N. sous les dénominations ALBUVIR (HC86, HC86 NK, HC90), par la Société ISIGNY sous la dénomination ISOPRO S 75.
- les protéines du babeurre telles que celles vendues par exemple par la Société ISIGNY sous la dénomination babeurre doux à 30% de matière active;
- les "protéines totales" du lait, obtenues à partir de lait écrémé.

De tels produits sont vendus par la Société DE MELKINDUSTRIE VEGHEL sous les dénominations MPC 75 et REFIT HPA ou par la Société ARMOR PROTEINES sous la dénomination LR 80F.

Conformément à la présente invention, on peut également utiliser les hydrolysats de ces différentes protéines.

Des hydrolysats de caséines sont vendus par exemple par la Société ARMOR PROTEINES sous la dénomination PEPTIDES N3, par la Société CRODA sous la dénomination HYDROLACTIN 2500.

Des hydrolysats de protéines de lactosérum sont vendus par la Société U.L.N. sous la dénomination SEROVIR HF, par la Société ARMOR PROTEINES sous la dénomination PEPTIDES 80.

Des hydrolysats de "protéines totales" sont vendus par la Société L.S.N. sous la dénomination LACTOLAN LS 5879 ou par la Société BROOKS sous la dénomination HYDROMILK 20.

Les protéines de lait et/ou leurs hydrolysats sont présents dans les compositions conformes à l'invention, dans des concentrations comprises de préférence entre 0,02 et 15% en poids par rapport au poids total de la composition.

Les hydrolysats de kératine sont présents dans les compositions selon l'invention dans des concentrations comprises de préférence entre 0,1 et 10% en poids par rapport au poids total de la composition.

Les compositions conformes à la présente invention peuvent se présenter sous forme de lotions aqueuses, hydroalcooliques, éventuellement épaissies ou des gels. Elles peuvent être conditionnées soit en flacon-pompe pour être appliquées sous forme de pulvérisation ou de spray, soit conditionnées en aérosol pour être appliquées sous forme de spray et de préférence sous forme de mousse.

Le milieu cosmétiquement acceptable peut être constitué par de l'eau et/ou un solvant organique cosmétiquement acceptable tel que plus particulièrement les monoalcools ayant de 1 à 8 atomes de carbone comme l'éthanol, l'isopropanol, l'alcool benzylique, l'alcool phényléthylique, les polyalcools tels que les alkylèneglycols comme l'éthylèneglycol, le propylèneglycol, utilisés seuls ou en mélanges. Le solvant est présent de préférence dans des proportions inférieures ou égales à 50% en poids par rapport au poids total de la composition et de préférence de 5 à 30%.

Les compositions conformes à la présente invention peuvent contenir des additifs habituellement utilisés en cosmétique, compatibles avec les protéines de lait ou leurs hydrolysats et les hydrolysats de kératine, choisis parmi les émollients, les lubrifiants, les agents pénétrants, les stabilisants, les parfums, les agents épaississants, les agents conservateurs et éventuellement les plastifiants, les agents cationiques, les polymères, les silicones ou d'autres protéines animales ou végétales éventuellement quaternisées.

Les épaississants, utilisés dans les compositions conformes à l'invention, sont choisis de préférence parmi les polymères d'acide acrylique réticulés ou non et plus particulièrement les acides polyacryliques réticulés par un agent polyfonctionnel tels que les produits vendus sous la dénomination CARBOPOL par la Société GOODRICH, les dérivés de cellulose tels que la méthylcellulose, l'hydroxyéthylcellulose, l'hydroxypropylméthylcellulose, les sels de sodium de la carboxyméthylcellulose ou les copolymères éthylène/anhydride maléïque de haut poids moléculaire.

Ces épaississants sont présents de préférence entre 0,05 et 5% et plus particulièrement entre 0,1 et 2% en poids par rapport au poids total de la composition.

Lorsque les compositions conformes à l'invention sont conditionnées en aérosol pour être appliquées sous forme de spray ou de mousse, les agents propulseurs peuvent être choisis parmi les hydrocarbures volatils tels que le n-butane, le propane, l'isobutane ou leurs mélanges ou un mélange de ces hydrocarbures avec des hydrocarbures chlorés et/ou fluorés tels que les composés vendus sous la dénomination de FREON ou DYMEL par la Société DU PONT DE NEMOURS.

Ils peuvent également être choisis parmi les hydrocarbures chlorés et/ou fluorés susdécrits et leurs mélanges, le gaz carbonique et le protoxyde d'azote.

On préfère, selon l'invention, utiliser un mélange de n-butane, isobutane, propane et de monofluorotrichlorométhane et plus particulièrement un mélange de n-butane, d'isobutane et de propane.

Dans les compositions de spray, la phase propulsive représente 20 à 80% du poids total de la composition pressurisée.

Dans les compositions de mousse aérosol, la phase propulsive représente de 2 à 15% du poids total de la composition pressurisée.

Les compositions conformes à l'invention sont utilisées pour la mise en forme ou pour la fixation des cheveux. Elles peuvent être appliquées comme produit de traitement après la coloration, décoloration, après le shampooing, après la permanente ou le défrisage des cheveux.

Lorsque les compositions selon l'invention sont utilisées pour la mise en forme de la chevelure, la concentration totale de protéine de lait ou son hydrolysat et d'hydrolysats de kératine est comprise de préférence entre 0,1 et 5% et plus particulièrement entre 0,2 et 2% en poids par rapport au poids total de la composition.

Lorsque les compositions sont utilisées pour la fixation des cheveux, la concentration totale des protéines de lait et/ou de leurs hydrolysats et d'hydrolysats de kératine est comprise de préférence entre 0,2 et 15% en poids et plus particulièrement entre 0,5 et 10% en poids par rapport au poids total de la composition.

Une composition particulièrement préférée selon l'invention est constituée par une composition de mise en forme des cheveux sous forme de mousse aérosol.

Le procédé de traitement cosmétique des cheveux destiné à favoriser leur maintien qui constitue un autre objet de l'invention, consiste à appliquer au moins une protéine de lait et/ou un hydrolysat de protéine de lait et au moins un hydrolysat de kératine dans un milieu cosmétiquement acceptable, sur les cheveux mouillés ou séchés, au moyen des compositions décrites ci-dessus.

Les exemples suivants sont destinés à illustrer l'invention.

Les dénominations commerciales mentionnées dans la description et les exemples désignent, sous toute réserve, des marques commerciales déposées.

### EXEMPLE 1

On prépare une mousse de coiffage de composition suivante :

| | |
|---|---|
| - Hydrolysat de kératine de PM 100.000, vendu en solution aqueuse à 15,4% de MA sous la dénomination KERASOL par la Société CRODA | 0,4 g MA |
| - Mélange (80/20) de caséine et de protéines du lactosérum, vendu à 89,5% de MA sous la dénomination REFIT HPA par la Société DE MELKINDUSTRIE VEGHEL | 0,27 g MA |
| - Hydrolysat de protéine de soja quaternisé, vendu à 30% de MA sous la dénomination CROQUAT SOYA par la Société CRODA | 0,3 g MA |
| - Parfum, colorant, conservateur qs | |
| - Eau déminéralisée | qsp 100 g |

| **Conditionnement aérosol** : | |
|---|---|
| - Composition ci-dessus : | 95 g |
| - Mélange ternaire de n-butane, isobutane > 55%, propane, vendu sous la dénomination AEROGAZ 3,2 N par la Société ELF AQUITAINE | 5 g |
| | TOTAL 100 g |

### EXEMPLE 2

On prépare un spray de fixation de composition suivante :

| | |
|---|---|
| - Caséinate de calcium vendu à 90,3% de MA sous la dénomination CALCIUM CASEINATE I par la Société DE MELKINDUSTRIE VEGHEL | 9,03 g MA |
| - Hydrolysat de kératine de PM 100.000, vendu en solution aqueuse à 15% de MA sous la dénomination KERASOL par la Société CRODA | 3 g MA |
| - Parfum, conservateur qs | |
| - Eau déminéralisée | qsp 100 g |

Cette composition est conditionnée dans un flacon pompe.

### EXEMPLE 3

On prépare un gel de coiffage de composition suivante :

| | |
|---|---|
| - Hydrolysat de kératine quaternisée, vendu en solution aqueuse à 30% de MA sous la dénomination CROQUAT WKP par la Société CRODA | 0,3 g MA |
| - Protéines de babeurre vendues sous la dénomination BABEURRE DOUX à 30% de MA par la Société ISIGNY | 0,06 g MA |
| - Acide polyacrylique réticulé, vendu sous la dénomination CARBOPOL 940 (PM 4.000.000) par la Société GOODRICH | 0,5 g |
| - Triéthanolamine qs pH=7 | |
| - Parfum, conservateur qs | |
| - Eau déminéralisée | qsp 100 g |

### EXEMPLE 4

On prépare une mousse de coiffage de composition suivante :

| | |
|---|---|
| - Hydrolysat de kératine de PM 100.000, vendu en solution aqueuse à 15% de MA sous la dénomination KERASOL par la Société CRODA | 0,5 g MA |
| - Caséinate de calcium | 0,3 g MA |
| - Parfum, colorant, conservateur qs | |
| - Eau déminéralisée | qsp 100 g |

| **Conditionnement aérosol** : | |
|---|---|
| - Composition ci-dessus : | 90 g |
| - Mélange ternaire de n-butane, isobutane > 55%, propane, vendu sous la dénomination AEROGAZ 3,2 N par la Société ELF AQUITAINE | 10 g |
| | TOTAL 100 g |

## Revendications

1. Composition cosmétique destinée au maintien de la chevelure, caractérisée par le fait qu'elle contient dans un milieu cosmétiquement acceptable, au moins une protéine de lait et/ou un hydrolysat de protéine de lait et au moins un hydrolysat de kératine de poids moléculaire compris entre 100 et 200.000.

2. Composition selon la revendication 1, caractérisée par le fait que l'hydrolysat de kératine est obtenu par hydrolyse de kératine issue des cheveux, de la laine, de la peau, des poils, de soies, de plumes, d'écailles, de sabots ou de come et peut comporter des groupements quaternaires greffés sur la chaîne peptidique ou en bout de chaîne.

3. Composition selon la revendication 1 ou 2, caractérisée par le fait que l'hydrolysat de kératine est choisi parmi :
(i) les hydrolysats de poids moléculaire d'environ 100.000 obtenus par hydrolyse modérée de kératine de sabots de bovin;
(ii) les hydrolysats de poids moléculaire d'environ 150 obtenus par hydrolyse acide ménagée;
(iii) les hydrolysats comportant sur leur chaîne peptidique des groupements ammonium quaternaires comprenant au moins une chaîne grasse en C₁₀-C₁₈.

4. Composition selon l'une quelconque des revendications 1 à 3, caractérisée par le fait que les protéines de lait sont choisies parmi les caséines et les protéines du lactosérum obtenues à partir de lait de vache ou de sous-produits du lait résultant de la fabrication du fromage ou du beurre, ou leurs hydrolysats.

5. Composition selon l'une quelconque des revendications 1 à 4, caractérisée par le fait que les protéines de lait sont choisies parmi :
(i) les caséines obtenues à partir de lait écrémé par précipitation, par voie acide, par fermentation lactique ou par de la presure, puis séchées, ou leurs hydrolysats;
(ii) les protéïnes du lactosérum, séparées du lactosérum par ultrafiltration, électrodialyse, chromatographie échangeuse d'ions, chromatographie d'exclusion par la taille, ou leurs hydrolysats;
(iii) les protéines du babeurre, ou leurs hydrolysats;
(iv) les protéines dites "totales" obtenues à partir de lait écrémé ou leurs hydrolysats;
ainsi que leurs mélanges.

6. Composition selon l'une quelconque des revendications 1 à 5, caractérisée par le fait qu'elle contient au moins de 0,02 à 15% en poids de protéine de lait et/ou d'hydrolysat de protéine de lait et au moins de 0,1 à 10% en poids d'hydrolysat de kératine, par rapport au poids total de la composition.

7. Composition selon l'une quelconque des revendications 1 à 6, caractérisée par le fait qu'elle se présente sous forme de lotion aqueuse ou hydroalcoolique, épaissie ou non ou sous forme de gel.

8. Composition selon l'une quelconque des revendications 1 à 7, caractérisée par le fait qu'elle est conditionnée sous forme de flacon-pompe pour être appliquée sous forme de pulvérisation ou de spray ou conditionnée en aérosol pour être appliquée sous forme de spray ou de mousse.

9. Composition selon l'une quelconque des revendications 1 à 8, caractérisée par le fait que le milieu cosmétiquement acceptable contient de l'eau et/ou un solvant organique choisi parmi les monoalcools ayant 1 à 8 atomes de carbone, les polyalcools utilisés seuls ou en mélanges, ledit solvant étant présent dans des proportions inférieures ou égales à 50% en poids par rapport au poids total de la composition.

10. Composition selon l'une quelconque des revendications 1 à 9, caractérisée par le fait qu'elle contient des additifs compatibles avec les protéines de lait, leurs hydrolysats et les hydrolysats de kératine choisis parmi les émollients, les lubrifiants, les agents pénétrants, les stabilisants, les parfums, les agents épaississants, les agents conservateurs, les plastifiants, les agents cationiques, les polymères, les silicones ou des protéines animales ou végétales éventuellement quaternisées, autres que celles définies dans les revendications 4 et 5.

11. Composition selon l'une quelconque des revendications 1 à 10, caractérisée par le fait qu'elle contient de 0,05 à 5% en poids et de préférence de 0,1 à 2% en poids par rapport au poids total de la composition, d'un agent épaississant choisi parmi les polymères d'acide acrylique réticulés ou non, les dérivés de cellulose ou les copolymères éthylène/anhydride maléïque de haut poids moléculaire.

12. Composition selon l'une quelconque des revendications 1 à 11, conditionnée en aérosol pour être appliquée sous forme de spray ou de mousse, caractérisée par le fait qu'elle contient un agent propulseur choisi parmi le n-butane, le propane, l'isobutane ou leurs mélanges ou un mélange de ces hydrocarbures avec des hydrocarbures chlorés et/ou fluorés; les hydrocarbures chlorés et/ou fluorés ou leurs mélanges; le gaz carbonique ou le protoxyde d'azote.

13. Composition selon la revendication 12, caractérisée par le fait qu'elle contient un agent propulseur choisi parmi un mélange de n-butane, d'isobutane, de propane et de monofluorotrichlorométhane ou un mélange de n-butane, propane et d'isobutane.

14. Composition conditionnée en aérosol pour être appliquée sous forme de spray, selon la revendication 12 ou 13, caractérisée par le fait qu'elle contient de 20 à 80% en poids d'agent propulseur par rapport au poids total de la composition.

15. Composition conditionnée en aérosol pour être appliquée sous forme de mousse selon la revendication 12 ou 13, caractérisée par le fait qu'elle contient de 2 à 15% en poids d'agent propulseur par rapport au poids total de la composition.

16. Composition selon l'une quelconque des revendications 1 à 15, caractérisée par le fait qu'elle est utilisée pour la mise en forme des cheveux ou la fixation des cheveux comme produit de traitement pouvant être appliqué après la coloration, décoloration, après le shampooing, après la permanente ou le défrisage des cheveux.

17. Composition selon la revendication 16, pour la mise en forme des cheveux, caractérisée par le fait que la concentration totale de protéine de lait et/ou d'hydrolysat de protéine de lait et d'hydrolysat de kératine est comprise entre 0,1 et 5% en poids par rapport au poids total de la composition.

18. Composition selon la revendication 16 pour la fixation des cheveux, caractérisée par le fait que la concentration totale de protéine de lait et/ou d'hydrolysat de protéine de lait et d'hydrolysat de kératine est comprise entre 0,2 et 15% en poids par rapport au poids total de la composition.

19. Procédé de traitement cosmétique des cheveux, destiné à favoriser leur maintien, caractérisé par le fait que l'on applique au moins une protéine de lait et/ou un hydrolysat de protéine de lait et au moins un hydrolysat de kératine de poids moléculaire compris entre 100 et 200.000, dans un milieu cosmétiquement acceptable sur les cheveux mouillés ou séchés, au moyen d'une composition telle que définie selon l'une quelconque des revendications 1 à 18.

## Claims

1. Cosmetic composition intended for holding the hair, characterized in that it contains, in a cosmetically acceptable medium, at least one milk protein and/or milk protein hydrolysate and at least one keratin hydrolysate of molecular weight between 100 and 200,000.

2. Composition according to claim 1, characterized in that the keratin hydrolysate is obtained by hydrolysis of keratin originating from hair, wool, skin, bristles, silks, feathers, scales, hooves or horn, and can contain quaternary groups grafted on the peptide chain or at the end of the chain.

3. Composition according to claim 1 or 2, characterized in that the keratin hydrolysate is chosen from:
(i) hydrolysates of molecular weight approximately 100,000 obtained by moderate hydrolysis of bovine hoof keratin;
(ii) hydrolysates of molecular weight approximately 150 obtained by controlled acid hydrolysis;
(iii) hydrolysates containing, on their peptide chain, quaternary ammonium groups comprising at least one C₁₀-C₁₈ fatty chain.

4. Composition according to any one of claims 1 to 3, characterized in that the milk proteins are chosen from caseins and whey proteins obtained from cow's milk or from milk by-products resulting from cheese- or buttermaking, or their hydrolysates.

5. Composition according to any one of claims 1 to 4, characterized in that the milk proteins are chosen from:
(i) caseins obtained from skimmed milk by precipitation, by acid treatment, by lactic fermentation or using rennet, and then dried, or their hydrolysates;
(ii) whey proteins, separated from whey by ultrafiltration, electrodialysis, ion exchange chromatography, size exclusion chromatography, or their hydrolysates;
(iii) buttermilk proteins or their hydrolysates;
(iv) so-called "total" proteins obtained from skimmed milk, or their hydrolysates;
as well as the mixtures thereof.

6. Composition according to any one of claims 1 to 5, characterized in that it contains at least from 0.02 to 15% by weight of milk protein and/or of milk protein hydrolysate and at least from 0.1 to 10% by weight of keratin hydrolysate, relative to the total weight of the composition.

7. Composition according to any one of claims 1 to 6, characterized in that it takes the form of an agueous or aqueous-alcoholic lotion, thickened or otherwise, or the form of a gel.

8. Composition according to any one of claims 1 to 7, characterized in that it is packaged in the form of a pump bottle for application in the form of an atomization or spray, or packaged as an aerosol for application in the form of a spray or mousse.

9. Composition according to any one of claims 1 to 8, characterized in that the cosmetically acceptable medium contains water and/or an organic solvent chosen from monohydric alcohols having 1 to 8 carbon atoms and polyhydric alcohols, used alone or mixed, said solvent being present in proportions not exceeding 50% by weight relative to the total weight of the composition.

10. Composition according to any one of claims 1 to 9, characterized in that it contains additives compatible with the milk proteins, their hydrolysates and the keratin hydrolysates, chosen from emollients, lubricants, penetrating agents, stabilizers, perfumes, thickening agents, preservatives, plasticizers, cationic agents, polymers, silicones or animal or vegetable proteins, optionally quaternized, other than those defined in claims 4 and 5.

11. Composition according to any one of claims 1 to 10, characterized in that it contains from 0.05 to 5% by weight, and preferably from 0.1 to 2% by weight, relative to the total weight of the composition, of a thickening agent chosen from acrylic acid polymers, crosslinked or otherwise, cellulose derivatives or high molecular weight ethylene/maleic anhydride copolymers.

12. Composition according to any one of claims 1 to 11, packaged as an aerosol for application in the form of a spray or mousse, characterized in that it contains a propellent agent chosen from n-butane, propane, isobutane or the mixtures thereof, or a mixture of these hydrocarbons with chlorinated and/or fluorinated hydrocarbons; chlorinated and/or fluorinated hydrocarbons or the mixtures thereof; carbon dioxide or nitrous oxide.

13. Composition according to claim 12, characterized in that it contains a propellent agent chosen from a mixture of n-butane, isobutane, propane and monofluorotrichloromethane, or a mixture of n-butane, propane and isobutane.

14. Composition packaged as an aerosol for application in the form of a spray, according to claim 12 or 13, characterized in that it contains from 20 to 80% by weight of propellent agent relative to the total weight of the composition.

15. Composition packaged as an aerosol for application in the form of a mousse, according to claim 12 or 13, characterized in that it contains from 2 to 15% by weight of propellent agent relative to the total weight of the composition.

16. Composition according to any one of claims 1 to 15, characterized in that it is used for the shaping of hair or the fixing of hair as a treatment product which can be applied after dyeing or bleaching, after shampooing or after permanent-waving or straightening of hair.

17. Composition according to claim 16 for the shaping of hair, characterized in that the total concentration of milk protein and/or of milk protein hydrolysate and of keratin hydrolysate is between 0.1 and 5% by weight relative to the total weight of the composition.

18. Composition according to claim 16 for the fixing of hair, characterized in that the total concentration of milk protein and/or of milk protein hydrolysate and of keratin hydrolysate is between 0.2 and 15% by weight relative to the total weight of the composition.

19. Process for the cosmetic treatment of hair, intended for promoting the holding thereof, characterized in that at least one milk protein and/or milk protein hydrolysate and at least one keratin hydrolysate of molecular weight between 100 and 200,000 are applied in a cosmetically acceptable medium to wet or dried hair, by means of a composition as defined according to any one of claims 1 to 18.

## Patentansprüche

1. Kosmetische Zusammensetzung zur Festlegung der Haare, dadurch **gekennzeichnet**, daß
sie in einem kosmetisch geeigneten Milieu mindestens ein Milchprotein und/oder ein Hydrolysat eines Milchproteins und mindestens ein Keratin-Hydrolysat eines Molekulargewichts von 100 bis 200000 enthält.

2. Zusammensetzung gemäß Anspruch 1,
dadurch **gekennzeichnet**, daß
das Keratin-Hydrolysat durch Hydrolyse von Keratin aus Haaren, Wolle, Haut, Tierhaaren, Seiden, Federn, Schildpatt, Hufen oder Horn erhältlich ist und quaternäre Ammoniumgruppen aufweisen kann, die auf die Peptidkette oder an deren Kettenende aufgepfropft sind.

3. Zusammensetzung gemäß Anspruch 1 oder 2,
dadurch **gekennzeichnet**, daß
das Hydrolysat von Keratin ausgewählt ist aus:
(i) Hydrolysaten eines Molekulargewichts von ca. 100000, erhältlich durch gemäßigte Hydrolyse von Keratin aus Rinderhufen;
(ii) Hydrolysaten eines Molekulargewichts von ca. 150, erhältlich durch saure Abbau-Hydrolyse;
(iii) Hydrolysaten, die auf ihrer Peptidkette quaternäre Ammoniumgruppen aufweisen, die mindestens eine C₁₀₋₁₈-Fettkette enthalten.

4. Zusammensetzung gemäß jedem der Ansprüche 1 bis 3,
dadurch **gekennzeichnet**, daß
die Milchproteine aus Kaseinen und Proteinen des Milchserum, welche aus Kuhmilch oder Nebenprodukten der Milch erhältlich sind, die bei der Herstellung von Käse oder Butter entstehen, oder aus deren Hydrolysaten ausgewählt sind.

5. Zusammensetzung gemäß jedem der Ansprüche 1 bis 4,
dadurch **gekennzeichnet**, daß
die Milchproteine ausgewählt sind aus:
(i) Kaseinen, erhältlich aus entrahmter Milch durch Ausfällung, auf saurem Weg, durch Milchfermentation oder Gerinnung und anschließende Trocknung, oder aus deren Hydrolysaten;
(ii) Proteinen des Milchserum, abgetrennt aus Lactoserum durch Ultrafiltration, Elektrodialyse, Ionenaustauschchromatographie oder Größenausschlußchromatographie, oder aus deren Hydrolysaten;
(iii) Proteinen der Buttermilch oder aus deren Hydrolysaten;
(iv) als "Gesamt" bezeichneten Proteinen, erhältlich aus entrahmter Milch, oder aus deren Hydrolysaten,
sowie ans deren Mischungen.

6. Zusammensetzung gemäß jedem der Ansprüche 1 bis 5,
dadurch **gekennzeichnet**, daß
sie mindestens 0,02 bis 15 Gew.% Milchprotein und/oder Hydrolysat von Milchprotein und mindestens 0,1 bis 10 Gew.% Hydrolysat von Keratin enthält, bezogen auf das Gesamtgewicht der Zusammensetzung.

7. Zusammensetzung gemäß jedem der Ansprüche 1 bis 6,
dadurch **gekennzeichnet**, daß
sie in Form einer wässrigen oder hydrolakoholischen, gegebenenfalls verdickten Lotion oder in Form eines Gel vorliegt.

8. Zusammensetzung gemäß jedem der Ansprüche 1 bis 7,
dadurch **gekennzeichnet**, daß
sie in Form einer Zusammensetzung für ein Pumpen-Fläschchen zur Aufbringung in Form eines Zerstäubungs- oder Sprayprodukts oder als Aerosol zur Aufbringung in Form eines Spray oder Schaums zubereitet ist.

9. Zusammensetzung gemäß jedem der Ansprüche 1 bis 8,
dadurch **gekennzeichnet**, daß
das kosmetisch geeignete Milieu Wasser und/oder ein organisches Lösungsmittel enthält, ausgewählt aus Niedrigalkoholen mit 1 bis 8 Kohlenstoffatomen oder aus Polyalkoholen, welche alleine oder in Mischungen verwendet werden, wobei das genannte Lösungsmittel in Mengenanteilen von weniger oder gleich 50 Gew.% vorhanden ist, bezogen auf das Gesamtgewicht der Zusammensetzung.

10. Zusammensetzung gemäß jedem der Ansprüche 1 bis 9,
dadurch **gekennzeichnet**, daß
sie Additive enthält, die mit den Milchproteinen, deren Hydrolysaten und den Keratin-Hydrolysaten verträglich und aus weichmachenden Mitteln, Gleitmitteln, Eindringmitteln, Stabilisiermitteln, Parfüm-Produkten, Verdickungsmitteln, Konservierungsmitteln, Plastifiziermitteln, kationischen Mitteln, Polymeren, Silikonen oder tierischen oder pflanzlichen, gegebenenfalls quaternierten Proteinen, die sich von den in den Ansprüchen 4 und 5 definierten unterscheiden, ausgewählt sind.

11. Zusammensetzung gemäß jedem der Ansprüche 1 bis 10,
dadurch **gekennzeichnet**, daß
sie 0,05 bis 5 und vorzugsweise 0,1 bis 2 Gew.%, bezogen auf das Gesamtgewicht der Zusammensetzung, eines Verdickungsmittels enthält, das aus gegebenenfalls vernetzten Acrylsäure-Polymeren, Cellulosederivaten oder aus Ethylen/Maleinanhydrid-Copolymeren eines hohen Molekulargewichts ausgewählt ist.

12. Zusammensetzung gemäß jedem der Ansprüche 1 bis 11, die als Aerosol zur Aufbringung in Form eines Spray oder Schaums zubereitet und
dadurch **gekennzeichnet** ist, daß sie ein
Treibmittel enthält, das aus n-Butan, Propan, Isobutan oder deren Mischungen oder aus einer Mischung dieser Kohlenwasserstoffe mit chlorierten und/oder fluorierten Kohlenwasserstoffen, aus chlorierten und/oder fluorierten Kohlenwasserstoffen oder deren Mischungen, aus Kohlensäuregas oder aus Distickstoffmonoxid ausgewählt ist.

13. Zusammensetzung gemaß Anspruch 12,
dadurch **gekennzeichnet**, daß
sie ein Treibmittel enthält, das aus einer Mischung aus n-Butan, Isobutan, Propan und Monofluortrichlormethan oder aus einer Mischung aus n-Butan, Propan und Isobutan ausgewählt ist.

14. Als Aerosol zubereitete Zusammensetzung gemäß Anspruch 12 oder 13 zur Aufbringung in Form eines Spray,
dadurch **gekennzeichnet**, daß
sie 20 bis 80 Gew.% Treibmittel enthält, bezogen auf das Gesamtgewicht der Zusammensetzung.

15. Als Aerosol zubereitete Zusammensetzung gemäß Anspruch 12 oder 13 zur Aufbringung in Form eines Schaums,
dadurch **gekennzeichnet**, daß
sie 2 bis 15 Gew.% Treibmittel enthält, bezogen auf das Gesamtgewicht der Zusammensetzung.

16. Zusammensetzung gemäß jedem der Ansprüche 1 bis 15,
dadurch **gekennzeichnet**, daß
sie zur Formgebung der Haare oder zur Fixierung der Haare als Produkt zur Behandlung verwendet wird, wobei sie nach einer Färbung, Entfärbung, Schamponierung, Dauerwelle oder Ausfrisierung der Haare aufgebracht werden kann.

17. Zusammensetzung gemäß Anspruch 16 zur Formgebung der Haare,
dadurch **gekennzeichnet**, daß
die Gesamtkonzentration an Milchprotein und/oder Hydrolysat davon und an Keratin-Hydrolysat 0,1 bis 5 Gew.% beträgt, bezogen auf das Gesamtgewicht der Zusammensetzung.

18. Zusammensetzung gemäß Anspruch 16 zur Fixierung der Haare,
dadurch **gekennzeichnet**, daß
die Gesamtkonzentration an Milchprotein und/oder Hydrolysat davon und an Keratin-Hydrolysat 0,2 bis 15 Gew.% beträgt, bezogen auf das Gesamtgewicht der Zusammensetzung.

19. Verfahren zur kosmetischen Behandlung der Haare zur Begünstigung von deren Halt bzw. Sitz,
dadurch **gekennzeichnet**, daß
man mindestens ein Milchprotein und/oder ein Hydrolysat von Milchprotein sowie mindestens ein Keratin-Hydrolysat eines Molekulargewichts von 100 bis 200000, in einem kosmetisch geeigneten Milieu, auf feuchte oder getrocknete Haare mittels einer in jedem der Ansprüche 1 bis 18 definierten Zusammensetzung aufbringt.
